# EUROPEAN PATENT APPLICATION

(11) **EP 2 380 628 A1**
(43) Date of publication of application: **26.10.2011**
(21) Application number: 10160489.0
(22) Date of filing: 20.04.2010
(51) Int. Cl.: A61N 2/02, A61N 1/40

(54) **Portable re-balancing unit for stimulating an organism electromagnetically**

(71) Applicant: HealthTech Sciences AS, 5008 Bergen (NO)
(72) Inventor: Aksnes, Thomas, 5310, Hauglandshella (NO)
(74) Representative: Lous, Carsten

(57) **Abstract**

An apparatus and method for transmitting an electromagnetic field that affects the bio-energetic field of an organism comprising a high frequency carrier wave that transmits low frequency information signals to aid in rebalancing said bio-energetic field. By using a high frequency and a low frequency electromagnetic field, with the addition of a scalar field, it is possible to provide a rebalancing device that is small, portable, easy to use and effective.

## Description

### Technical Field

The present invention relates to an apparatus and method for generating electromagnetic (EM) frequencies for the stimulation and rebalancing of living organisms.

### Background

As current research has shown living organisms produce a characteristic vibration pattern that consists of low frequency EM (electro-magnetic) vibration. This is typically referred to as a bio-energetic field. It has been determined that one indication of the state of health of an organism can be determined by measuring these fields. Moreover, experimentation has shown that affecting these fields in the right way can have a beneficial effect on the state of health of an organism. A further explanation of how a disturbance in the bio-energetic field can affect health is given in the detailed description below.

Recognising this link between the bio-energetic field and health has led to a variety of devices being developed to rebalance or correct this field. One such device is known from WO95/01750. Using this device involves attaching up to 24 electrodes to a person along meridians of their body. These electrodes then measure the body's energy allowing a trained person to make a health assessment diagnosis. This device claims to also apply a therapeutic treatment to the body, either by using these same electrodes to apply an electromagnetic field to specific points on the body, or by using specially designed massage electrodes which vibrate using a computer controlled solenoid. It is also suggested that the electromagnetic field generated by the solenoid's winding may itself have a therapeutic effect. The device of WO95/01750 is quite a sophisticated unit and can be programmed to address various imbalances in an individual. However the unit is quite large, so the patient has to remain stationary while treatment is taking place. It also requires operation by a trained individual. Attaching the large number of electrodes is cumbersome and time consuming, and also requires specialist knowledge to know where to place them. A unit of this type is also quite expensive to manufacture, and is too costly for the average individual to buy for personal use.

Another device is known from WO02/26321, which describes a small wearable therapeutic bio-energy unit. It is a passive unit that relies upon the existing energy in the body or in the surroundings, interacting with the specially shaped coils to achieve an effect. These units are designed to be built into a variety of items such as carpet, clothing, furniture, steering wheels etc. Also described is the possibility of using what are termed electromagnetic correctors together with the bio-energy units. These correctors can be battery powered and microprocessor controlled. The purpose of these correctors is to provide a shielding magnetic field with a chosen distribution of intensity and direction. In WO02/26321 the bio-energy unit is quite small and can be worn by the individual, but being a passive unit its effectiveness is limited. It is also not possible to adapt or program it in any way to suit a particular imbalance of an individual. Mention is made of microprocessor controlled magnetic correctors and although details of their construction or function are completely absent, it appears from the description that they are designed to have a shielding effect.

So there exists a need for a unit that is adaptable to individual imbalances, easy and convenient to use, small and portable, affordable for the average individual to buy for personal use, and efficient at conveying information to an organism with the intent of restoring health creating patterns, at the same time as being completely safe.

### Disclosure of the Invention

The invention relates to a method and apparatus to generate electromagnetic fields for correcting and stimulating the bio-energetic field of an organism, and aims at solving the problems of the prior art devices which are either too large, heavy, expensive, cumbersome and require extensive training to use, or if small enough are very limited in their functionality (i.e. not adaptable to specific patients needs) and effectiveness (being of small size prior art devices have very limited ability to affect the bio-energetic field). Furthermore the PRU is more effective than larger devices at affecting the bio-energetic field due to its sophisticated means of generating and transmitting EM waves.

The invention consists of an apparatus for generating an electromagnetic field which is applied to the bio-energetic field of an organism, characterised in that it comprises a means for generating a high frequency carrier wave of between 1 MHz and 1 GHz, with a preferred frequency of 10MHz, and means to modulate said carrier wave by a low frequency signal of between 0.1 and 200Hz and preferably between 0.2 and 99.9Hz.

This apparatus will be referred to in the rest of the text as a Portable Rebalancing Unit (PRU). Advantageously, using the high frequency carrier wave to transmit the low frequency information signal, results in a greater efficiency and effectiveness than using either of said frequencies independently. What is meant by this is that the effect on the bio-energetic field is greater when using the HF and LF together. This unexpected effect is occurring because of the interaction between the HF/LF waves and the bio-energetic field and not because of an interaction between the HF and LF waves themselves. A resulting advantage of this greater efficiency and effectiveness is that less energy is expended in applying the electromagnetic waves, which thereby makes it possible to not only miniaturize the PRU but also to have a long battery life. So the synergy between the high frequency and low frequency waves achieves an effect beyond that of using either wave alone even at far higher signal strengths. In general applying the low frequency information signal to a high frequency carrier wave in the MHz or GHz range requires less power than using a low frequency information signal on its own (i.e. without a carrier wave). It is emphasized that the lower power requirement occurs because the information being sent to the bio-energetic field is being effectively received at lower field strengths. Using the specific frequency of 10MHz has the advantages that:
(i) It is effective as a carrier wave for the simple signals that need to be sent (of low frequency therefore low data density).
(ii) The frequency of 10MHz directly stimulates cellular activity.
(iii) 10MH is the best frequency for generating strong scalar fields.
(iv) 10MHz is good for the short distances needed to reach the users body without affecting other people nearby.

A further aspect of the invention consists in an apparatus comprising a means for amplitude modulating the carrier wave using means to generate programmed sequences of said low frequency signals, wherein said signals are of variable frequency, cycles, program length and duty ratio, and wherein said apparatus comprises means for storing said programmed sequences and a user interface to select said sequences. Advantageously, by selecting the parameters of the aforementioned variables, it is possible to target systems and processes within an organism. By allowing the program selection to be carried out by the user, the unit can be adapted to their specific imbalances or needs. Amplitude modulation of the carrier wave has been tested as being more efficient.

A further aspect of the apparatus is that it comprises a user interface adapted to modify or add to the stored sequences. This has the advantage of allowing the unit to be updated with the most recent and relevant programs as well as allowing the user to create their own program sequences.

A further aspect of the invention consists in an apparatus wherein the means for applying said electromagnetic field to an organism is comprised of a coil antenna. Advantageously, the use of a coil antenna allows the signals to be transferred to an organism without requiring galvanic skin contact. Additional advantages due to the non-contact nature of the PRU are that it does not need to be placed in a specific location, such as on an acupuncture point or along a meridian, making it easier to use and also allows it to be easily carried in a convenient location on the body chosen by the user. A further advantage is that it allows EM waves produced by the unit to reach further into the body of an organism, as the signals are electromagnetically transmitted by the coil antenna and not just applied to the surface of the skin. As the coil is contained within the unit it can be made compact, robust and self contained.

A further aspect of the invention consists in an apparatus wherein the number of turns of the coil is 100 to 200 and preferably 150, the inner diameter of the coil is 12mm to 30mm and preferably 19mm and the outer diameter of the coil 14mm to 35mm and preferably 23mm, and the thickness of the wire is 0.1 mm to 0.4mm and preferably 0.2mm. The effect of using this type of coil is that a low power secondary field in addition to the main field is generated, wherein said secondary field is an exact phase inversion of the main field, and wherein the main field is comprised of the modulated carrier wave. Advantageously, the combination of this secondary field with the main field creates a scalar wave (standing wave). The body has been shown to be transparent (i.e. unaffected) by the scalar wave itself, but the scalar wave is however very effective at carrying information which can be received by the body. Using this type of coil has the advantage of increasing the efficiency of applying information signals to the body while minimising the power required.

### Brief Description of the Drawings

Fig. 1 shows a diagram of the functional parts of the PRU
Fig. 2 shows the waves produced by the coil
Fig. 3 is a diagram of the structural elements of the coil

### Detailed Description

When a body manifests ill health, there can be many reasons for this. It has been shown that there are measurable changes in the bio-energetic field when an individual (organism) is unwell. One approach at understanding those imbalances is to consider that the organism's self healing capacity has been compromised in some way - or put another way; it lacks the information required to bring itself back into balance. An example of the information we are talking about, is the characteristic frequency of an organ. Many processes are taking place in an organ at any given time and there are many different frequencies present at the cellular, intercellular and micro-cellular level, as explained later in this document. However there is a general or characteristic frequency, which can be measured in a healthy organ and which is believed to bring some order to the chaos of the huge number of complex activities going on in an organ or system. Weakness in organs and imbalance in processes are linked to disrupted information, and the approach of the PRU is to provide information which enables the self regulating mechanisms to engage processes that can manifest in improving weakness and imbalance.

So the PRU aims at providing informational support in the form of carefully chosen frequencies, to aid the self regulating process. When this self regulating process is disturbed it may give rise to a dys-regulation or what is commonly experienced by an organism as a symptom. One example of the result of such a dys-regulation could be high blood pressure or a headache. The PRU is designed to focus on the self regulation process behind defined and localised issues. The PRU is not treating either the aforementioned potential manifestation (symptom) or the physiology associated with its manifestation or prevention, but provides information to activate specific aspects of self regulation within the organism's body that may be disturbed. When the information flow is disturbed or disrupted, this can result in a general disturbance that will display in a currently weak area, and can thus work its way through to a manifestation (via a weakness in the self regulating system). An example of this could be an individual with a less than optimal lifestyle, who has developed a weakness in the digestive system due to bad diet. When he/she is also exposed to microorganisms, extra stress at work, or something else, the body's information system is already functioning at reduced capacity, and the proper mechanisms to deal with these extra factors may not be set in motion sufficiently. These new factors then create symptomatic problems, and in addition there is probably a symptomatic worsening of the chronic imbalances, like indigestion. When the PRU provides the information of how to deal with all these factors, the self regulating mechanisms can solve the problems more easily, and the chronic imbalances can also be improved.

Sometimes the self regulating system is sufficiently strong and/or supported by the body system and signs of a disturbance do not occur. Sometimes the body is strong enough that disturbances are minimal i.e. its physical resources alone can overcome the informational disturbances. However a lot of the time this does not happen and we need to offer support to restore wellness.

The PRU cooperates with our inherent, informational healing programs. It can be viewed as a finely-tuned assistant to boost our own perfect ability to heal. The PRU influences our frequency-based information system and tailors its manner of interaction according to each specific health condition.

The PRU works with the body on an electromagnetic level. It uses the understanding of how the body is influenced through communication energetically. All cells are molecules, and molecules generate electromagnetic frequencies based on their subatomic vibration. These frequencies are an important part of the cell to cell communication and guidance of the body's self-regulating mechanisms. When a health condition appears, the molecular movements change, and by exposing them to the correct frequencies, they will have the opportunity to re-tune themselves back to their normal state.

A simple example of this can be described as follows. Organs and systems in the body have been shown to have a characteristic frequency, for example 6Hz, which assists in regulation of the parasympathetic system. A malfunctioning organ or system is not in tune with this frequency. As explained in the beginning of the detailed description, this characteristic frequency is believed to help to achieve some order in the chaos of the many activities going on, thereby reducing the entropy and leading to better alignment and harmony of these activities. To aid the correction of this imbalance as quickly and efficiently as possible the high frequency of the PRU is used to stimulate the cellular activity while at the same time the low frequency is used to achieve bio-resonance of the specific organ or system.

It is important to note that due to the informational aspect of the signal that is sent to the body, there is a built in safety mechanism. If a program is run that is incorrect for the imbalance being addressed, then the body has no need of that information and does not respond to the signal.

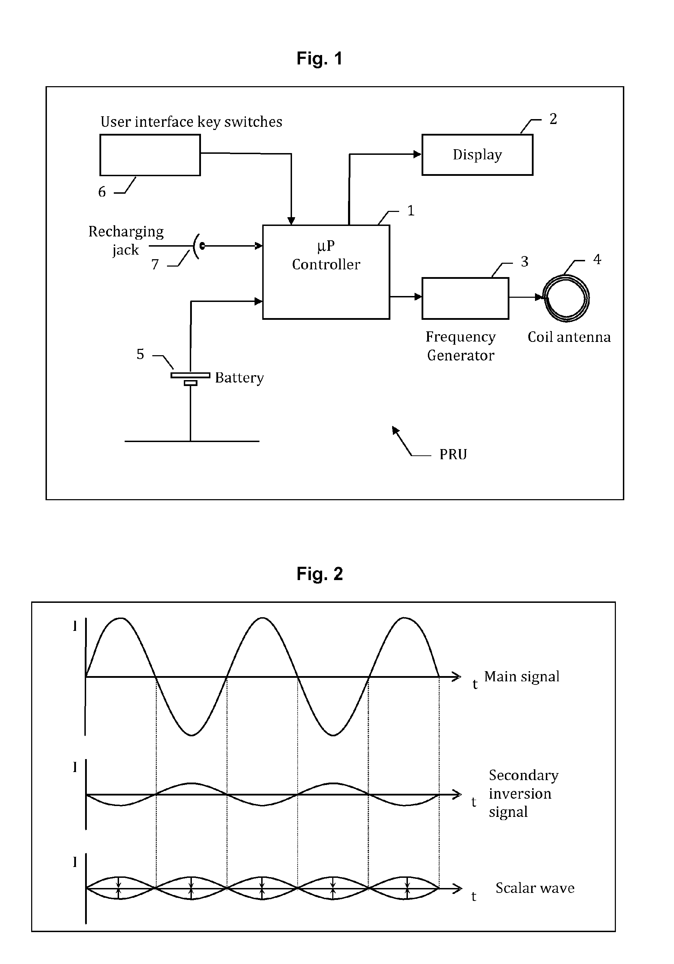

Figure 1 shows the functional parts of the PRU. A microprocessor 1 controls the coil drive unit 3 to generate output signals which drive the coil 4. Also connected to the microprocessor controller 1 are a display 2 and a user key interface 6. Power is supplied by a rechargeable battery 5. The battery is recharged through the recharging jack 7.The coil drive unit includes a frequency generator which is controlled by the microprocessor 1 and sends a sequence of low frequency waveforms of variable duty ratio, frequency and amplitude by amplitude modulating the high frequency carrier wave. These waveforms are digitally generated sine waves. A user selects the desired program using the key interface and the display interface. These interfaces are well known to a person skilled in the art and may consist of various types of keys such as push button, membrane, soft touch or touch screen and various types of display screen such as LCD, OLED, ink style (as used in e-book readers) or LED. When the desired program has been selected it is displayed on the screen and then activated. When the program has finished the PRU will switch off automatically. The key interface also allows the user to create their own programs by setting the frequency, duty ratio, cycles and program length and other parameters manually. Of these parameters cycles refers to the number of time a program segment is repeated. The other parameters are self evident. Advantageously this reprogramming is done without the need for any external devices such as cables or computers.

An aspect of the invention which aids the efficiency and effectiveness of the PRU, is the creation of scalar waves along with the HF and LF waves. The scalar waves are generated by an interaction of the HF wave with a phase inverted copy of itself. In Figure 2 can be seen a diagram which shows this process. A secondary wave is generated due to the structural nature of the coil antenna, which generates this secondary wave which is phase inverted with respect to the signal wave (main wave) fed to the antenna. Due to the exact phase inversion, the current of each wave is cancelled out. This results in a scalar wave which is a wave with no electrical field strength but still carries information. Further information about scalar waves and their ability to carry and transmit information is available in the publication of Professor Konstantin Meyl: "Electromagnetic Environmental Compatibility", INDEL Verlagsabteilung Villingen-Schwenningen, 1st Edition 2003, ISBN 3-9802 542-4-0 2004 Kinokuniya Company, LTD (see http://www.tfcbooks.com/mall/more/610sw.htm for a summary of the book).

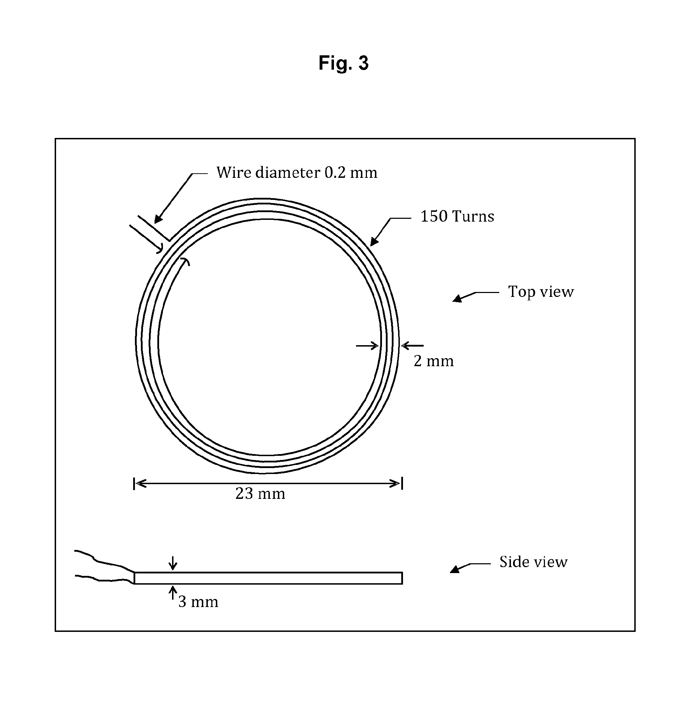

The generated waves are applied to the body using a compact coil antenna. This allows the waves to penetrate clothing and the surface of the skin to reach areas where the waves can have the greatest effect while the compactness of the antenna is one of the features which allows the unit to be of a small size. Figure 3 shows the dimensions and structure of the coil antenna used for 10MHz. The dimensions of the coil were arrived at by iterative experimentation and selected to achieve an exact phase inversion at the modulated carrier wave frequency. The field strength generated by this coil antenna when running a typical program varies from about 80 milligauss next to the coil to about 1.8 milligauss at a distance of 20cm from the coil.

According to a first embodiment, the method of the invention can be described as the use of a high frequency carrier wave for transmitting low frequency cell rebalancing signals. It is known that changes can be made on the cellular level by stimulating the cells with certain electromagnetic frequencies. Some computer driven bio-energetic devices use high frequencies (above 1 MHZ). These high frequencies (HF's) are known to stimulate or trigger cell activity. However to make something specific happen it is necessary to use much lower frequencies (LF's) for example 0.5-100Hz. However low frequencies are not as effective at reaching the cells, so a much higher power is needed to ensure that the cells have the ability to be affected by these low frequency signals. So by using the high frequency to carry the low frequency information to the, much less power is needed. This is due to several factors, one of which is that the receptivity of the cells is increased by stimulating them with specific HF's, such as 10MHz.

An additional effect that occurs when using the combination of HF and LF is that the effect of the LF is greater than when the LF is applied without a carrier wave irrespective of the power of the LF wave being used. This effect has been tested by using a non specific HF carrier wave for example 900MHz, a carrier wave which in itself has no effect on the cell activity. It was noticed that the effects of the LF were better and faster than using the LF alone. So there are two positive effects that are achieved - one by using a carrier wave and one by using a carrier wave of a specific frequency that stimulates cells. Various other tests were carried out to determine the effects of the high frequency. Using two separate signal generators, one for LF and the other for HF, it was shown that the presence of an HF signal concurrently with the LF signal also had a positive synergetic effect compared to using the same HF and LF with an hour between them. So it is clear that there are several effects that result from the combined use of HF and LF signals. These effects are noticeable even when the HF and LF are applied concurrently by separate generators, but are much stronger when using the HF as a carrier wave for the LF, and stronger still when specific HF frequencies are used such as 10MHz. It is possible to use higher frequencies up to the GHz range but it was found that 10MH proved to be the most efficient and practical for the reasons listed on page 3.

According to another embodiment, the effectiveness of the PRU is enhanced by the use of a coil antenna to radiate the HF and LF electromagnetic (EM) field signals. This antenna uses a specific number of turns and a specific diameter so that a part of the signal is phase inverted compared to the main signal. This results in part of the signal is cancelled out. At the point that the weak signal is at a negative peak, the main signal has a positive peak, so the amplitude of the weak signal is cancelled out. This results in no amperage in the signal, which means that the EM field is absent leaving only a scalar field. There are some aspects of the body that are more sensitive to scalar fields than to magnetic fields. Again there is a synergistic effect that arises from the combination of an EM field with a scalar field that enhances the effectiveness that each type of field has on its own i.e. the sum of information from the two fields is reaching more aspects of the targets of influence. The scalar field that is generated has the same frequency as the EM field. This is because of the use of the coil antenna which is tuned by selecting the number of turns to achieve an exact phase inversion, which results in the generation of both scalar and EM fields.

According to another embodiment, the electric and magnetic parts of the EM field have been fine tuned to be below the threshold where any stress response is noticed in the body. This means that there is no stress response, but the effect is at a maximum because the organism always responds better when only positive /harmonious activities are stimulated. The electric properties of the PRU's EM field are consequently very weak, and the magnetic field is much stronger. The body is much more tolerant of magnetic fields than electric fields.

According to another embodiment, is a method of running programs in the PRU wherein the selection of the order of the program steps is as follows: A set program starts by running a preparatory sub-program followed by the main program. An example of this is the detox program. First the sub-program is run which optimises the organs that are involved in the detox process such as the kidney, liver and lymphatic system then the program stimulates the dissolving of various toxins like heavy metals, solvents etc., followed finally by the stimulation of the detox process. So in this example there are three stages: the body is enabled to handle the process, toxins are dissolved, and then lastly the detox process is kick-started. This sequencing of sub-programs is done automatically in the PRU, so the user does not need knowledge about the required steps. In prior art machines, if these types of programs are available, the sequencing has to be done manually.

According to another embodiment the PRU can be contained in a volume of less than 100cm3. The efficiency of the carefully chosen (in some cases custom made) and matched components, the synergistic effect of the specific HF and LF, and the use of a coil antenna all combine to make possible the miniaturization of what the prior art has needed laptop sized computers, ancillary items, high power and various probes etc. to achieve. The advantage of miniaturization is that it allows personal use on a daily basis as it can be carried unobtrusively. Another advantage is that it costs a fraction of the larger stationary devices as fewer components are needed, and in many cases the PRU provides better results because it can be used regularly (on a daily basis), without requiring a visit to a clinic where such devices and their practitioners are located.

The effectiveness of the PRU can be demonstrated using an EIS device. The EIS (Electro Interstitial Scan) is approved in USA, Europe and several other countries as a medical device for monitoring the effects of any treatment.

A sample of some of the many tests done showing the effect of the PRU can be seen below:

### Case Example of EIS 5 Day Test:

Test Person: Male 61 year old. Feels ok, but white blood cell count is low, and there are some minor complaints about digestive system and respiratory system. First EIS test results are in the left column together with the date of the test. Here the EIS shows a percentage below normal functional ability of the state of the tested organ or system. Some variation in organ activity is normal during the day, and also from day to day, but major (greater than 20%) negative percentage shifts show a problem. A reduction in the negative percentage shows an improvement in that organ or system.

In this case the EIS indicates reduced cellular activity in the whole digestive system, as well as the respiratory system. The date and time of the measurements are next to the percentage modulations, so only 5 days have passed in-between these measurements. The test itself is 96% reproducible. The only change the client made in these 5 days was to include daily use of 3 PRU programs:
1) "Regulation of digestive organs"
2) "Regulation of Lungs / Bronchi"
3) "Regulation of Adrenal Gland".

| **Digestive system.** Program used: "Regulation of digestive organs", once a day for 5 days | | |
|---|---|---|
| Esophagus | 17.08.09: -33% | 22.08.09: -11% |
| Stomach | 17.08.09: -33% | 22.08.09: -11% |
| Duodenum | 17.08.09: -33% | 22.08.09: -11% |
| Small intestine | 17.08.09: -45% | 22.08.09: -18% |
| Ceacum | 17.08.09: -34% | 22.08.09: -14% |
| Ascending colon | 17.08.09: -34% | 22.08.09: -14% |
| Transversal colon by liver | 17.08.09: -37% | 22.08.09: -19% |
| Transversal colon by spleen | 17.08.09: -37% | 22.08.09: -14% |
| Descending colon | 17.08.09: -45% | 22.08.09: -21% |
| Sigmoid colon | 17.08.09: -45% | 22.08.09: -21% |
| Liver, right lobe | 17.08.09: -30% | 22.08.09: -21 % |
| Liver, left lobe | 17.08.09: -38% | 22.08.09: -21 % |
| Pancreas | 17.08.09: -29% | 22.08.09: -07% |

| **Respiratory system.** Program used: "Regulation of Lungs / Bronchi", once a day for 5 days | | |
|---|---|---|
| Trachea and upper bronchi | 17.08.09: -27% | 22.08.09: -11% |
| Upper lobe of right bronchi/lung | 17.08.09: -35% | 22.08.09: -14% |
| Middle lobe of right bronchi/lung | 17.08.09: -37% | 22.08.09: -17% |
| Lower lobe of right bronchi/lung | 17.08.09: -39% | 22.08.09: -20% |
| Upper lobe of left bronchi/lung | 17.08.09: -23% | 22.08.09: -04% |
| Lower lobe of left bronchi/lung | 17.08.09: -36% | 22.08.09: -19% |

| **Adrenals**. Program used: "Regulation of Adrenal Gland", once a day for 5 days. | | |
|---|---|---|
| Right adrenocortical | 17.08.09: -37% | 22.08.09: -20% |
| Right medullo-adrenal | 17.08.09: -40% | 22.08.09: -21% |
| Left adrenocortical | 17.08.09: -37% | 22.08.09: -20% |
| Left medullo-adrenal | 17.08.09: -40% | 22.08.09: -21% |

The above test shows significant improvements in the readings for the tested organs and systems due to the use of the PRU over a five day period. The EIS also has a graphic output which indicates clearly the location and degree of improvement indicated by the numbers listed above. It is also possible to demonstrate that the PRU can have an effect in the short term as well.

### Case Example of EIS 30 Minute Test:

Patient 48 years old, reporting several unresolved emotional factors. The EIS test showed reduced activity in the hypothalamus, which can result in psychosomatic regulatory problems. The PRU program "Regulation of Hypothalamus / Pituitary" was used once with time reduced to half of the default. An EIS test was performed right before and right after running the program. An imbalance of cellular functional activity at the level of 24% reduced to 13% in less than half an hour. This is however a temporary balancing, which generally will only hold for hours or days unless the causation is also dealt with.

In summary, the key difference between the PRU and prior art devices is the combined use of specific HF and LF signals which leads to greater effectiveness than using either HF or LF signals independently. Furthermore using this combination achieves this result without inducing any stress response even if the PRU is used continuously for long periods of time i.e. for more than several uninterrupted hours on a daily basis. This makes the PRU both safe and extremely effective.

## Claims

1. An apparatus for generating an electromagnetic field which is applied to the bio-energetic field of an organism, **characterised in that** it comprises a means for generating a high frequency carrier wave of between 1 MHz and 1 GHz, with a preferred frequency of 10MHz, and means to modulate said carrier wave by a low frequency signal of between 0.1 and 200Hz and preferably 0.2 and 99.9Hz.

2. An apparatus according to claim 1 wherein it comprises a means for amplitude modulating the carrier wave using means to generate programmed sequences of said low frequency signals, wherein said signals are of variable frequency, cycles, program length and duty ratio, and wherein said apparatus comprises means for storing said programmed sequences and a user interface to select said sequences.

3. An apparatus according to claim 2 wherein it comprises a user interface adapted to modify or add to the stored sequences.

4. An apparatus according to claims 1 or 2 comprising means for generating and transmitting a scalar wave concurrently with the LF and HF waves.

5. An apparatus according to claim 4 wherein the means for applying said electromagnetic field to an organism is comprised of a coil antenna.

6. An apparatus according to claim 5 wherein the number of turns of the coil is 100 to 200 and preferably 150, the inner diameter of the coil is 12mm to 30mm and preferably 19mm and the outer diameter of the coil is 14mm to 60mm and preferably 23mm, and the thickness of the wire is 0.1 mm to 0.5mm and preferably 0.2mm.

7. A method for generating electromagnetic frequencies for the stimulation and rebalancing of the bio energetic field of living organisms **characterised by** using a high frequency carrier wave of between 1 MHz and 1 GHz for transmitting low frequency based information signals of 0.1 -200Hz to an organism via said bio energetic field.

8. A method according to claim 7 wherein said high frequency carrier wave is between 9 MHz and 11 MHz and preferably 10MHz and the low frequency signal is 0.2 -99.9 Hz.

9. A method according to claim 7 or 8 wherein said high frequency carrier wave is amplitude modulated by the low frequency signal.

10. A method according to claim 7, 8 or 9 comprising the step of adding a scalar wave to the HF and LF waves.

11. A method according to claim 7, 8, 9 or 10 wherein the low frequency based signals are transmitted in a programmed sequence of signals that have variable frequency, duty ratio, cycles and program length.
